# EUROPEAN PATENT APPLICATION

(11) **EP 3 666 765 A1**
(43) Date of publication of application: **17.06.2020**
(21) Application number: 18382909.2
(22) Date of filing: 11.12.2018
(51) Int. Cl.: C07D 311/74, A61P 25/00, A61P 35/00, A61K 31/353

(54) **CHROMENIC PHYTOCANNABINOIDS, THEIR SYNTHESIS AND USE IN TREATMENT OR PREVENTION OF DISEASE**

(71) Applicant: Emerald Health Biotechnology España,S.L.U., 14014 Córdoba (ES)
(72) Inventor: Muñoz Blanco, Eduardo, 14004 Córdoba (ES); Appendino, Giovanni, 10131 Torino (IT)
(74) Representative: Manuel Illescas y Asociados, S.L.

(57) **Abstract**

The present invention relates to a compound of the formula (I) or a solvate or co-crystal thereof 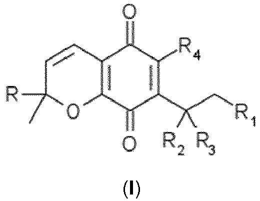 wherein:
- R is selected from the group consisting of: a C₁-C₆ alkyl, a C₂-C₈ alkenyl moiety, a C₄-C₁₂ dienyl or a C₆-C18 trienyl moiety;
- R₁ is a C₁-C₆ alkyl moiety;
- R₂ is selected from the group consisting of: H and a C₁-C₆ alkyl moiety;
- R₃ is selected from the group consisting of: H and a C₁-C₆ alkyl moiety;
- R₄ is selected from the group consisting of: H, a C₁-C₆ alkyl moiety and a benzyl moiety. The present invention also relates to a process for producing said compound of the formula (I), or a solvate or co-crystal thereof, as well as a pharmaceutical composition comprising said compound of the formula (I), or a solvate or co-crystal thereof and at least one excipient. The present invention also relates to a compound of the formula (I), or a solvate or co-crystal thereof, for use in the treatment or prevention of a disease or condition.

## Description

### Field of the invention

The present invention relates to the pharmaceutical field, namely to cannabinoids useful in the treatment or prevention of disease, and in particular to the treatment or prevention of a disease or condition responsive to inhibition of monoacylglycerol lipase (MAGL).

### Background to the invention

The endogenous cannabinoid system comprises endocannabinoids and two cannabinoid receptor subtypes, CB₁ and CB₂, which are involved in regulating many physiological processes. The CB₁ cannabinoid receptor is expressed principally in the central nervous system, but also in the lungs, liver and kidneys, while the CB₂ receptor is expressed principally in the immune system and in hematopoietic cells. Endocannabinoids are endogenous lipid-based retrograde neurotransmitters that bind to said cannabinoid receptors, of which 2-arachidonoylglycerol is an endocannabinoid that is an endogenous agonist of the CB₁ cannabinoid receptor and the primary endogenous ligand for the CB₂ cannabinoid receptor. Deactivation of 2-arachidonoylglycerol is principally performed by the monoacylglycerol lipase (MAGL) enzyme, which hydrolyses this endocannabinoid.

Thus, MAGL is involved in regulation of the levels of 2-arachidonoylglycerol and, thus, the activation and deactivation of the aforementioned cannabinoid receptor subtypes. Consequently, MAGL is also involved in regulation of physiological processes, diseases and conditions associated with the endogenous cannabinoid system. Said diseases and conditions comprise pain, cancer, neuroinflammation, neurodegeneration, amnesia, metabolic disease, obesity, diabetes, liver steatosis, anxiety, depression, stress, nausea, drug withdrawal, drug addiction, inflammation, convulsions, seizures, epilepsy and status epilepticus, amongst others (cf. https://doi.org/10.1016/j.bcp.2018.07.036). Said physiological processes comprise neuroprotection, cognitive improvements such as increased learning and memory capabilities, as well as feeding behaviour or appetite suppression.

It is a problem of the present invention to provide a compound for treatment or prevention of a disease or condition responsive to monoacylglycerol lipase inhibition. It is therefore also a problem of the present invention to provide a compound for treatment or prevention of a disease or condition selected from the group consisting of: pain, cancer, neuroinflammation, neurodegeneration, amnesia, metabolic disease, obesity, diabetes, liver steatosis, anxiety, depression, stress, nausea, drug withdrawal, drug addiction, inflammation, convulsions, seizures, epilepsy and status epilepticus. It is also a problem of the present invention to provide a compound for increasing neuroprotection, cognitive processes, learning, memory and feeding behaviour, or to suppress appetite.

It is a further problem of the present invention to provide a method of synthesis of a compound for treatment or prevention of a disease or condition responsive to monoacylglycerol lipase inhibition

### Brief description of the invention

The present invention relates to a compound of the formula (I) or a solvate or co-crystal thereof 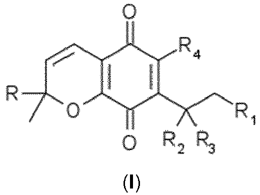 wherein:
- R is selected from the group consisting of: a C₁-C₆ alkyl, a C₂-C₈ alkenyl moiety, a C₄-C₁₂ dienyl or a C₆-C₁₈ trienyl moiety;
- R₁ is a C₁-C₆ alkyl moiety;
- R₂ is selected from the group consisting of: H and a C₁-C₆ alkyl moiety;
- R₃ is selected from the group consisting of: H and a C₁-C₆ alkyl moiety;
- R₄ is selected from the group consisting of: H, a C₁-C₆ alkyl moiety and a benzyl moiety.

The present invention also relates to a compound of the formula (I) or a solvate or co-crystal thereof 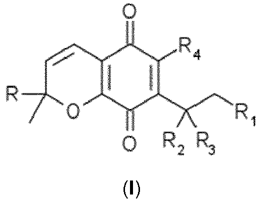 wherein:
wherein
- R is selected from the group consisting of: -CH₃, -CH₂-CH₂-CH=C(CH₃)₂, -CH₂-CH₂-CH=C(CH₃)-CH₂-CH₂-CH=C(CH₃)₂ and -CH₂-CH₂-CH=C(CH₃)-CH₂-CH₂-CH=C(CH₃)-CH₂-CH₂-CH=C(CH₃)₂;
- R₁ is selected from the group consisting of: -CH₃, -CH₂-CH₃, -CH₂-CH₂-CH₃, -CH₂-CH₂-CH₂-CH₃ and -CH₂-CH₂-CH₂-CH₂-CH₃;
- R₂ is selected from the group consisting of: H and CH₃;
- R₃ is selected from the group consisting of: H and CH₃; and
- R₄ is selected from the group consisting of: H, CH₃ and a benzyl moiety,
and wherein R₂ and R₃ are the same.

The present invention also relates to a process for producing a compound of the formula (I), or a solvate or co-crystal thereof 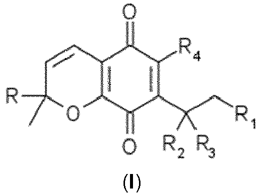 which comprises reacting a compound of formula (II), or a salt, solvate or co-crystal thereof 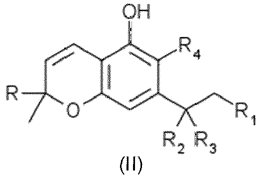 with a hypervalent iodine compound,
wherein:
- R is selected from the group consisting of: a C₁-C₆ alkyl, a C₂-C₈ alkenyl moiety, a C₄-C₁₂ dienyl or a C₆-C₁₈ trienyl moiety;
- R₁ is a C₁-C₆ alkyl moiety;
- R₂ is selected from the group consisting of: H and a C₁-C₆ alkyl moiety;
- R₃ is selected from the group consisting of: H and a C₁-C₆ alkyl moiety;
- R₄ is selected from the group consisting of: H, a C₁-C₆ alkyl moiety and a benzyl moiety.

The present invention also relates to a process for producing a compound of the formula (I) or a solvate or co-crystal thereof 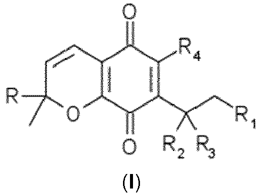 which comprises reacting a compound of formula (II), or a salt, solvate or co-crystal thereof 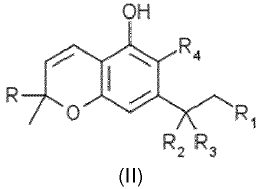 with a hypervalent iodine compound selected from the group consisting of: 2-iodoxybenzoic acid, 2-iodoxybenzoic acid stabilised with a mixture of benzoic acid and isophthalic acid in a molar ratio of 47:23:30, and 1,1,1-triacetoxy-1,1-dihydro-1,2-benziodoxol-3(1H)-one,
wherein:
wherein
- R is selected from the group consisting of: -CH₃, -CH₂-CH₂-CH=C(CH₃)₂, -CH₂-CH₂-CH=C(CH₃)-CH₂-CH₂-CH=C(CH₃)₂ and -CH₂-CH₂-CH=C(CH₃)-CH₂-CH₂-CH=C(CH₃)-CH₂-CH₂-CH=C(CH₃)₂;
- R₁ is selected from the group consisting of: -CH₃, -CH₂-CH₃, -CH₂-CH₂-CH₃, -CH₂-CH₂-CH₂-CH₃ and -CH₂-CH₂-CH₂-CH₂-CH₃;
- R₂ is selected from the group consisting of: H and CH₃;
- R₃ is selected from the group consisting of: H and CH₃; and
- R₄ is selected from the group consisting of: H, CH₃ and a benzyl moiety,
and wherein R₂ and R₃ are the same.

The present invention also relates to an intermediate compound in said process or steps prior thereto, wherein said compound is compound of formula (II), or a salt, solvate or co-crystal thereof 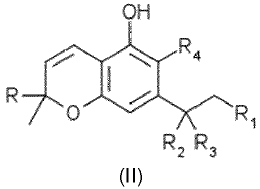 wherein:
- R is selected from the group consisting of: a C₁-C₆ alkyl, a C₂-C₈ alkenyl moiety, a C₄-C₁₂ dienyl or a C₆-C₁₈ trienyl moiety;
- R₁ is a C₁-C₆ alkyl moiety;
- R₂ is selected from the group consisting of: H and a C₁-C₆ alkyl moiety;
- R₃ is selected from the group consisting of: H and a C₁-C₆ alkyl moiety;
- R₄ is selected from the group consisting of: H, a C₁-C₆ alkyl moiety, a benzyl moiety and a -CHR₅-(4-morpholinyl) moiety,
wherein R₅ is selected from the group consisting of: H, a C₁-C₆ alkyl moiety and a phenyl moiety with the proviso that when R is -CH₂-CH₂-CH=C(CH₃)₂, R₂ is H, R₃ is H and R₄ is H, R₁ is not -CH₃ or -CH₂-CH₂-CH₃.

In addition, the present invention relates to a pharmaceutical or nutraceutical composition comprising said compound of the formula (I) or a solvate or co-crystal thereof, and at least one excipient.

Moreover, the present invention relates to said compound of the formula (I) or a solvate or co-crystal thereof, for use in the treatment or prevention of a disease or condition.

Furthermore, the present invention relates to said compound of the formula (I), or a solvate or co-crystal thereof, for use in the treatment or prevention of a disease or condition responsive to monoacylglycerol lipase inhibition, as well as to (i) use of said compound of the formula (I), or a solvate or co-crystal thereof, for the manufacture of a medicament for the treatment or prevention of a disease or condition responsive to monoacylglycerol lipase inhibition, (ii) a method of treatment or prevention of a disease or condition responsive to monoacylglycerol lipase inhibition in a subject, comprising administration of said compound of the formula (I), or a solvate or co-crystal thereof, to said subject, and (iii) a pharmaceutical or nutraceutical composition for the treatment or prevention of a disease or condition responsive to monoacylglycerol lipase inhibition comprising said compound of the formula (I), or a solvate or co-crystal thereof.

The present invention also relates to a compound of the formula (I) or a solvate or co-crystal thereof 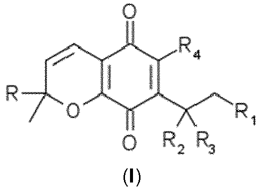 for use in the treatment or prevention of a disease or condition responsive to monoacylglycerol lipase inhibition, wherein said disease or condition is selected from the group consisting of: pain, cancer, neuroinflammation, neurodegeneration, amnesia, metabolic disease, obesity, diabetes, liver steatosis, anxiety, depression, stress, nausea, drug withdrawal, drug addiction, inflammation, convulsions, seizures, epilepsy and status epilepticus, wherein:
- R is selected from the group consisting of: -CH₃, -CH₂-CH₂-CH=C(CH₃)₂, -CH₂-CH₂-CH=C(CH₃)-CH₂-CH₂-CH=C(CH₃)₂ and -CH₂-CH₂-CH=C(CH₃)-CH₂-CH₂-CH=C(CH₃)-CH₂-CH₂-CH=C(CH₃)₂;
- R₁ is selected from the group consisting of: -CH₃, -CH₂-CH₃, -CH₂-CH₂-CH₃, -CH₂-CH₂-CH₂-CH₃ and -CH₂-CH₂-CH₂-CH₂-CH₃;
- R₂ is selected from the group consisting of: H and CH₃;
- R₃ is selected from the group consisting of: H and CH₃; and
- R₄ is selected from the group consisting of: H, CH₃ and a benzyl moiety,
and wherein R₂ and R₃ are the same.

### Figures

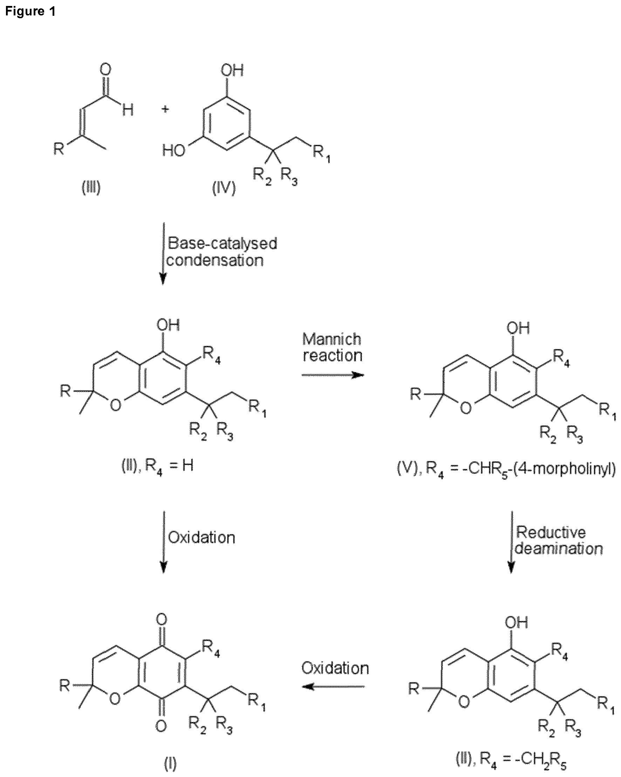

Figure 1: General synthetic scheme for synthesis of compounds of formula (I) of the present invention, when R₄ = H or when R₄ = CH₂R₅, as well as compounds of formula (II) of the present invention.

### Detailed description of the invention

The present invention relates to a compound of the formula (I) or a solvate or co-crystal thereof 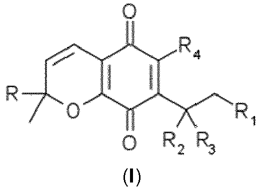 as well as to a process for producing said compound of the formula (I), or a solvate or co-crystal thereof, which comprises reacting a compound of formula (II), or a salt, solvate or co-crystal thereof 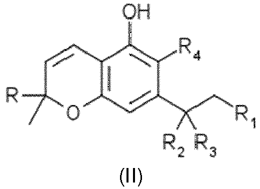 with a hypervalent iodine compound,
wherein:
- R is selected from the group consisting of: a C₁-C₆ alkyl, a C₂-C₈ alkenyl moiety, a C₄-C₁₂ dienyl or a C₆-C₁₈ trienyl moiety;
- R₁ is a C₁-C₆ alkyl moiety;
- R₂ is selected from the group consisting of: H and a C₁-C₆ alkyl moiety;
- R₃ is selected from the group consisting of: H and a C₁-C₆ alkyl moiety;
- R₄ is selected from the group consisting of: H, a C₁-C₆ alkyl moiety and a benzyl moiety.

The present invention also relates to a pharmaceutical or nutraceutical composition comprising said compound of the formula (I), or a solvate or co-crystal thereof, and at least one excipient. In addition, the present invention relates to said compound of the formula (I), or a solvate or co-crystal thereof for use in the treatment or prevention of a disease or condition. Moreover, the present invention relates to said compound of the formula (I), or a solvate or co-crystal thereof, for use in the treatment or prevention of a disease or condition responsive to monoacylglycerol lipase inhibition, as well as to (i) use of said compound of the formula (I), or a solvate or co-crystal thereof, for the manufacture of a medicament for the treatment or prevention of a disease or condition responsive to monoacylglycerol lipase inhibition, (ii) a method of treatment or prevention of a disease or condition responsive to monoacylglycerol lipase inhibition in a subject, comprising administration of said compound of the formula (I), or a solvate or co-crystal thereof, to said subject, and (iii) a pharmaceutical or nutraceutical composition for the treatment or prevention of a disease or condition responsive to monoacylglycerol lipase inhibition comprising said compound of the formula (I), or a solvate or co-crystal thereof.

In an embodiment of the present invention,
- R is selected from the group consisting of: a C₁-C₆ alkyl, a C₄-C₈ alkenyl moiety, a C₈-C₁₂ dienyl or a C₁₄-C₁₈ trienyl moiety;
- R₁ is a C₁-C₅ alkyl moiety;
- R₂ is selected from the group consisting of: H and a C₁-C₆ alkyl moiety;
- R₃ is selected from the group consisting of: H and a C₁-C₆ alkyl moiety; and
- R₄ is selected from the group consisting of: H, a C₁-C₆ alkyl moiety and a benzyl moiety,
and wherein R₂ and R₃ are the same.

In a preferred embodiment of the present invention,
- R is selected from the group consisting of: -CH₃, -CH₂-CH₂-CH=C(CH₃)₂, -CH₂-CH₂-CH=C(CH₃)-CH₂-CH₂-CH=C(CH₃)₂ and -CH₂-CH₂-CH=C(CH₃)-CH₂-CH₂-CH=C(CH₃)-CH₂-CH₂-CH=C(CH₃)₂;
- R₁ is selected from the group consisting of: -CH₃ and -CH₂-CH₂-CH₃.
- R₂ is selected from the group consisting of: H and CH₃;
- R₃ is selected from the group consisting of: H and CH₃; and
- R₄ is selected from the group consisting of: H, CH₃ and a benzyl moiety,
and wherein R₂ and R₃ are the same.

In a more preferred embodiment of the present invention, said compound of formula (I) is selected from the group consisting of: compounds (A), (B), (C), (D), (E), (F), (G) and (H):

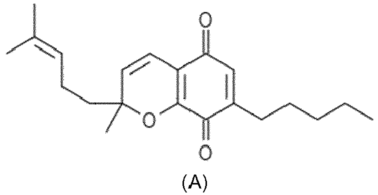

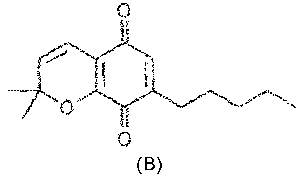

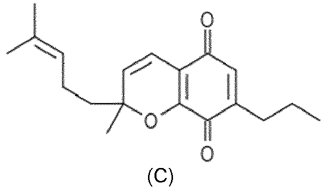

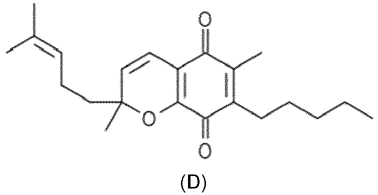

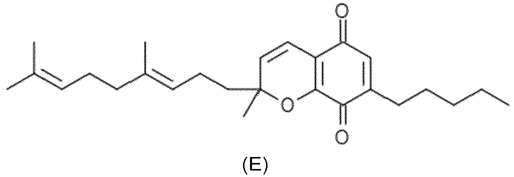

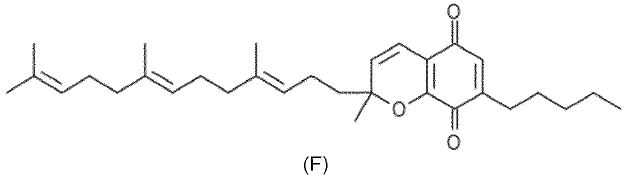

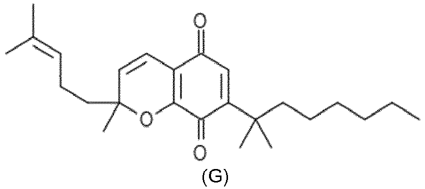

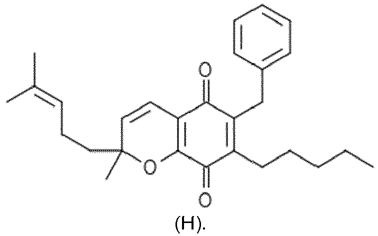

The process for producing said compound of the formula (I), or a solvate or co-crystal thereof, comprises an oxidation reaction (Figure 1). In a more preferred embodiment of the process of the present invention, said compound of formula (I) is selected from the group consisting of: compounds (A), (B), (C), (D), (E), (F), (G) and (H), as disclosed herein, wherein:
- when said compound of formula (I) is compound (A), the compound of formula (II) is compound (A') 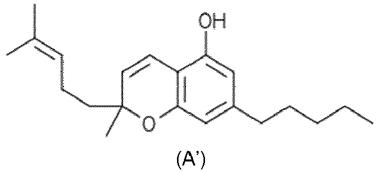
- when said compound of formula (I) is compound (B), the compound of formula (II) is compound (B') 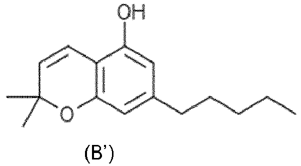
- when said compound of formula (I) is compound (C), the compound of formula (II) is compound (C') 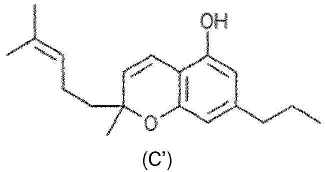
- when said compound of formula (I) is compound (D), the compound of formula (II) is compound (D') 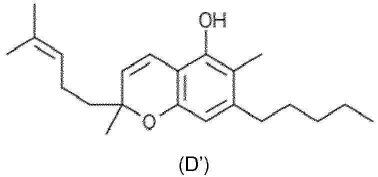
- when said compound of formula (I) is compound (E), the compound of formula (II) is compound (E') 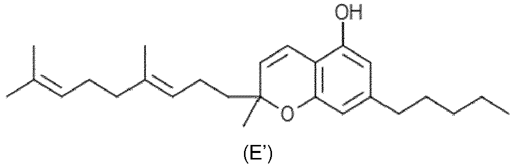
- when said compound of formula (I) is compound (F), the compound of formula (II) is compound (F') 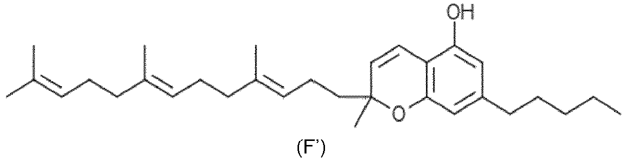
- when said compound of formula (I) is compound (G), the compound of formula (II) is compound (G') 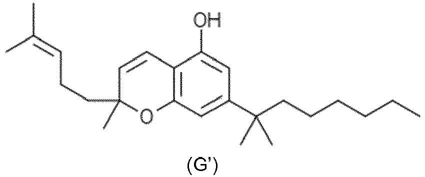
- when said compound of formula (I) is compound (H), the compound of formula (II) is compound (H')

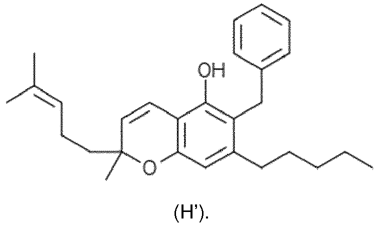

In an even more preferred embodiment of the present invention, said compound of formula (I) is selected from the group consisting of: compounds (A), (B) and (C):

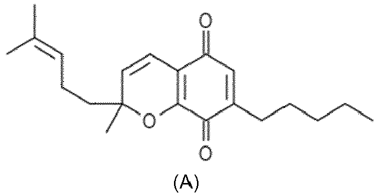

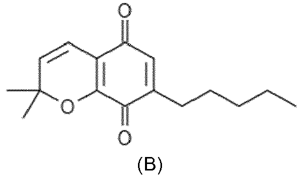

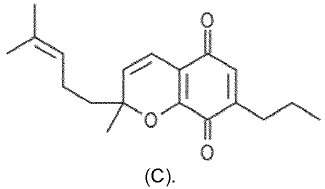

In an even more preferred embodiment of the process of the present invention, said compound of formula (I) is selected from the group consisting of: compounds (A), (B) and (C) wherein:
- when said compound of formula (I) is compound (A), the compound of formula (II) is compound (A')
- when said compound of formula (I) is compound (B), the compound of formula (II) is compound (B')
- when said compound of formula (I) is compound (C), the compound of formula (II) is compound (C').

In the present invention, all stereogenic double bonds in group R preferably have the E-configuration (trans-configuration). In addition, the compounds of formula (I), formula (II), formula (IV) and formula (V) which have a chiral centre may be enantiopure or comprise mixtures of enantiomers (including racemic mixtures) with respect to said chiral centre,

In the process for producing said compound of the formula (I), or a solvate or co-crystal thereof, of the present invention, the hypervalent iodine compound (oxidant) is preferably selected from the group consisting of: 2-iodoxybenzoic acid (IBX), 2-iodoxybenzoic acid stabilised with a mixture of benzoic acid and isophthalic acid in a molar ratio range of from 40:30:30 to 50:20:30, IBX on silica-gel, polymer-bound IBX (polystyrene-IBX) and 1,1,1-triacetoxy-1,1-dihydro-1,2-benziodoxol-3(1H)-one (Dess-Martin periodinane). More preferably, the hypervalent iodine compound is 2-iodoxybenzoic acid stabilised with a mixture of benzoic acid and isophthalic acid in a molar ratio range of from 45:25:30 to 49:21:30, even more preferably in a molar ratio of 47:23:30. As an alternative to the process for producing said compound of the formula (I), or a solvate or co-crystal thereof, of the present invention, is an equivalent process wherein a chromium (IV) compound or an oxoammonium-catalyst may be used as an oxidant instead of the hypervalent iodine compound, Preferably said chromium (IV) compound is selected from the group consisting of pyridinium chlorochromate, pyridinium dichromate and chromic oxide-pyridine (Collins reagent), while said oxoammonium-catalyst is selected from the oxoammonium salt formed by oxidation of a radical selected from the group consisting of TEMPO [(2,2,6,6-tetramethylpiperidin-1-yl)oxyl], 4-hydroxy-TEMPO, 4-amino-TEMPO, 4-oxo-TEMPO, 4-acetamido-TEMPO, 4-carboxy-TEMPO, TEMPO on silica gel, polymer-bound TEMPO (polystyrene-TEMPO), 2,2,6,6-tetramethyl-4-[1-oxo-6-(triethylammonio)hexylamino]-1-piperidinyloxy bromide (water-soluble TEMPO) and TEMPO immobilised on iron oxide (Fe₃O₄) superparamagnetic nanoparticles (TurboBeads™ TEMPO), wherein the oxidation of said radical is carried out in the presence of sodium hypochlorite, potassium hypochlorite, calcium hypochlorite or bis(acetoxy)iodobenzene.

Said oxidation reaction is preferably performed at between -20 and +30 °C, more preferably between 0 and 25 °C, even more preferably between 17 and 23 °C in a non-protic solvent selected from the group consisting of ethyl acetate, diethyl ether, tetrahydrofuran, dichloromethane and chloroform, still more preferably, ethyl acetate.

Thus, the present invention also relates to an intermediate compound in said process for producing said compound of the formula (I), or a solvate or co-crystal thereof, or steps prior thereto, wherein said intermediate compound is a compound of formula (II), or a salt, solvate or co-crystal thereof 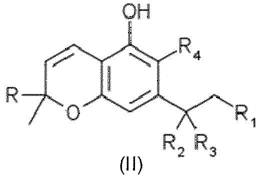 wherein:
- R is selected from the group consisting of: a C₁-C₆ alkyl, a C₂-C₈ alkenyl moiety, a C₄-C₁₂ dienyl or a C₆-C₁₈ trienyl moiety;
- R₁ is a C₁-C₆ alkyl moiety;
- R₂ is selected from the group consisting of: H and a C₁-C₆ alkyl moiety;
- R₃ is selected from the group consisting of: H and a C₁-C₆ alkyl moiety;
- R₄ is selected from the group consisting of: H, a C₁-C₆ alkyl moiety, a benzyl moiety, a formyl moiety and a -CHR₅-(4-morpholinyl) moiety,
   wherein R₅ is selected from the group consisting of: H, a C₁-C₆ alkyl moiety and a phenyl moiety with the proviso that when R is -CH₂-CH₂-CH=C(CH₃)₂, R₂ is H, R₃ is H, and R₄ is H, R₁ is not -CH₃ or -CH₂-CH₂-CH₃.

In a preferred embodiment of said intermediate compound,
- R is selected from the group consisting of: a C₁-C₆ alkyl, a C₄-C₈ alkenyl moiety, a C₈-C₁₂ dienyl or a C₁₄-C₁₈ trienyl moiety;
- R₁ is a C₁-C₅ alkyl moiety;
- R₂ is selected from the group consisting of: H and a C₁-C₆ alkyl moiety;
- R₃ is selected from the group consisting of: H and a C₁-C₆ alkyl moiety; and
- R₄ is selected from the group consisting of: H, a C₁-C₆ alkyl moiety, a benzyl moiety and a -CHR₅-(4-morpholinyl) moiety,
   wherein R₅ is selected from the group consisting of: H, a C₁-C₆ alkyl moiety and a phenyl moiety, wherein R₂ and R₃ are the same.

In a more preferred embodiment of said intermediate compound,
- R is selected from the group consisting of: -CH₃, -CH₂-CH₂-CH=C(CH₃)₂, -CH₂-CH₂-CH=C(CH₃)-CH₂-CH₂-CH=C(CH₃)₂ and -CH₂-CH₂-CH=C(CH₃)-CH₂-CH₂-CH=C(CH₃)-CH₂-CH₂-CH=C(CH₃)₂;
   - R₁ is selected from the group consisting of: -CH₃ and -CH₂-CH₂-CH₃.
- R₂ is selected from the group consisting of: H and CH₃;
- R₃ is selected from the group consisting of: H and CH₃; and
- R₄ is selected from the group consisting of: H, CH₃, a benzyl moiety and a -CHR₅-(4-morpholinyl) moiety, wherein R₅ is selected from the group consisting of: H and a phenyl moiety,
wherein R₂ and R₃ are the same.

In a still more preferred embodiment of said intermediate compound, said compound of formula (II) is selected from the group consisting of: compounds (A'), (B'), (C'), (D'), (E'), (F'), (G') and (H'):

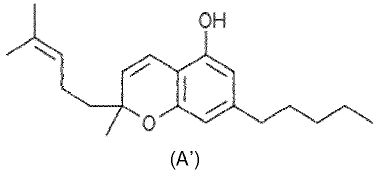

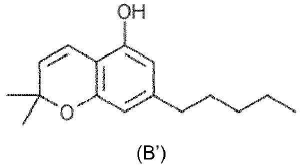

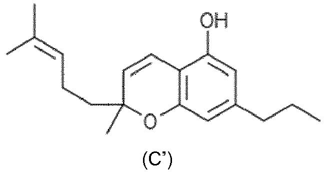

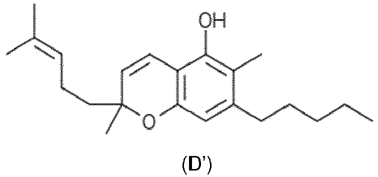

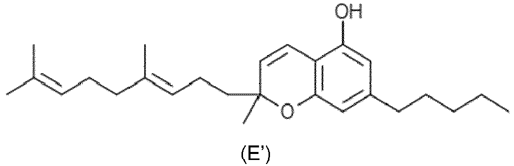

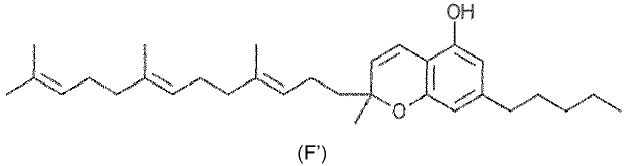

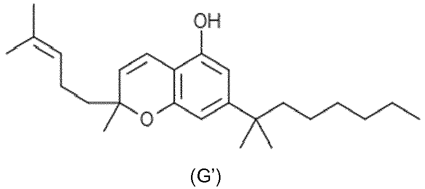

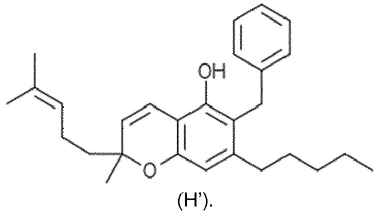

In an even more preferred embodiment of said intermediate compound, said compound of formula (II) is selected from the group consisting of: compounds (A'), (B') and (C'):

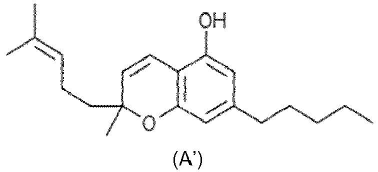

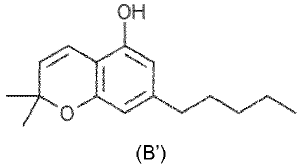

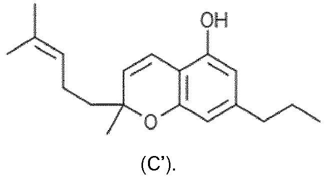

In one particularly preferred embodiment of the process of the present invention, when R₄ is H [such as in compounds (A) and (A'), (B) and (B'), (C) and (C'), (E) and (E'), (F) and (F') and (G) and (G')] the compound of formula (II) is formed by reacting a compound of formula (III) 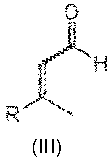 with a compound of formula (IV) 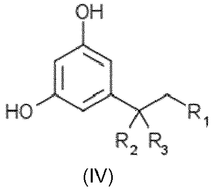 in the presence of a primary amine. Said reaction is a base-catalysed condensation (Figure 1), whereby said amine is said base.

In said base-catalysed condensation, said amine is preferably at least one primary alkyl amine or secondary alkyl amine or pyridine, more preferably at least one primary C₁-C₆ alkyl amine or pyridine, even more preferably said primary C₁-C₆ alkyl amine is selected from the group consisting of: ethylamine, *n*-propylamine, *iso*-propylamine, *n-*butylamine, *iso*-butylamine, *sec*-butylamine and *tert*-butylamine, still more preferably from the group consisting of: *n*-butylamine, *iso*-butylamine, *sec*-butylamine and *tert*-butylamine.

Said base-catalysed condensation is yet more preferably carried out in an aprotic solvent, preferably selected from the group consisting of: toluene, benzene, tetrahydrofuran, ether, dioxane, dichloromethane and chloroform, more preferably toluene, at a temperature greater than 50 °C, still more preferably at a temperature greater than 100 °C, between but less than 150 °C.

In another particularly preferred embodiment of the process of the present invention, when R₄ is a C₁-C₆ alkyl moiety of the formula -CH₂-R₅ or a benzyl moiety of the formula -CH₂R₅ [such as in compounds (D) and (D') or (H) and (H'), respectively] the compound of formula (II) is formed by reacting a compound of formula (V) 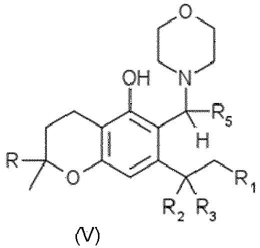 with a borohydride salt
wherein R₅ is selected from the group consisting of: H, a C₁-C₆ alkyl moiety and a phenyl moiety, and
- when R₅ is H, R₄ is a C₁ alkyl moiety;
- when R₅ is -CH₃, R₄ is a C₂ alkyl moiety;
- when R₅ is a C₂ alkyl moiety, R₄ is a C₃ alkyl moiety;
- when R₅ is a C₃ alkyl moiety, R₄ is a C₄ alkyl moiety;
- when R₅ is a C₄ alkyl moiety, R₄ is a C₅ alkyl moiety;
- when R₅ is a C₅ alkyl moiety, R₄ is a C₆ alkyl moiety; and
- when R₅ is a phenyl moiety, R₄ is a benzyl moiety. This reaction is a reductive deamination (Figure 1).

In said reductive deamination, the borohydride salt is preferably selected from the group consisting of: sodium cyanoborohydride, lithium cyanoborohydride, potassium cyanoborohydride, sodium borohydride, lithium borohydride, potassium borohydride, tetrabutylammonium borohydride, sodium triethylborohydride, lithium triethylborohydride and potassium triethylborohydride, more preferably from the group consisting of: sodium cyanoborohydride, lithium cyanoborohydride, sodium borohydride, and lithium borohydride, even more preferably, said borohydride salt is sodium cyanoborohydride.

Said reductive deamination is preferably performed in a solvent at a temperature greater than 50 °C, more preferably in a solvent which is at least one alcohol selected from the group consisting of: methanol, ethanol, *n-*propanol, *iso*-propanol, *n*-butanol, *iso*-butanol, *sec*-butanol and *tert*-butanol at a temperature greater than 60 °C, even more preferably in *n*-butanol, *iso*-butanol, *sec*-butanol or *tert*-butanol at a temperature greater than 100 °C but less than 130 °C.

In a more particularly preferred embodiment of the process of the present invention, said compound of formula (V) is formed by reacting a compound of formula (II) in which R₄ is H with a compound formed by reaction of morpholine with a compound of formula (VI) 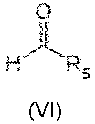 wherein R₅ is the same in the compound of formula (V) and the compound of formula (VI). This reaction is a Mannich reaction called a Betti reaction and the compound formed by reaction of morpholine with a compound of formula (VI) is a Mannich reagent.

Said Betti reaction is preferably performed at a temperature greater than 50 °C, more preferably at a temperature greater than 100 °C, even more preferably at a temperature greater than 120 °C but less than 130 °C.

In the present invention, alkyl moiety refers to monovalent group of empirical formula CₙH₍₂ₙ₊₁₎, alkenyl moiety refers to monovalent group of empirical formula CₙH₍₂ₙ₋₁₎, dienyl moiety refers to monovalent group of empirical formula CₙH₍₂ₙ₋₃₎, and trienyl moiety refers to monovalent group of empirical formula CₙH₍₂ₙ₋₅₎, which are respectively formed from the corresponding alkane, alkene, diene and triene, by removal of one hydrogen atom from any one of the n carbon atoms thereof.

The compounds of formula (I) of the present invention inhibit monoacylglycerol lipase (MAGL). Thus, the present invention also relates to use of a compound of the formula (I), or a solvate or co-crystal thereof, for inhibiting monoacylglycerol lipase.

In addition, the present invention also relates to a compound of the formula (I), or a solvate or co-crystal thereof, for the treatment or prevention of a disease or condition. The present invention also relates to a compound of the formula (I), or a solvate or co-crystal thereof, for the treatment or prevention of a disease or condition responsive to inhibition of MAGL. Analogously, the present invention also relates to (i) use of a compound of the formula (I), or a solvate or co-crystal thereof, for the manufacture of a medicament for the treatment or prevention of a disease or condition responsive to inhibition of MAGL (ii) a method of treatment or prevention of a disease or condition in a subject, comprising administration of a compound of the formula (I), or a solvate or co-crystal thereof, to said subject, wherein said disease or condition responsive to inhibition of MAGL, and (iii) a pharmaceutical or nutraceutical composition for the treatment or prevention of a disease or condition comprising a compound of the formula (I), or a solvate or co-crystal thereof, wherein said disease or condition responsive to inhibition of MAGL. Alternative to said treatment or prevention of a disease or condition, the compound of the formula (I), or a solvate or co-crystal thereof, may be used to increase neuroprotection, cognitive processes, learning, memory and feeding behaviour, or to suppress appetite.

In one embodiment of the present invention, said disease or condition responsive to inhibition of MAGL is selected from the group consisting of: pain, cancer, neurodegeneration, neuroinflammation, amnesia, metabolic disease, obesity, diabetes, liver steatosis, anxiety, depression, stress, nausea, drug withdrawal, drug addiction, inflammation, convulsions, seizures, epilepsy and status epilepticus. Preferably, said disease or condition responsive to inhibition of MAGL is selected from the group consisting of: pain, cancer, neurodegeneration, neuroinflammation, metabolic disease, obesity, diabetes, anxiety and depression.

In a preferred embodiment, said disease or condition responsive to inhibition of MAGL is pain, wherein said pain is more preferably selected from the group consisting of: nociceptive pain, neuropathic pain and psychogenic pain, still more preferably nociceptive pain selected from the group consisting of: visceral pain, deep somatic pain and superficial somatic pain, or neuropathic pain selected from the group consisting of: allodynia, mechanical allodynia, thermal allodynia, movement allodynia and phantom pain. In another preferred embodiment, said disease or condition responsive to inhibition of MAGL is a cancer selected from the group consisting of: breast cancer, lung cancer, prostate cancer, colorectal cancer, melanoma, pancreatic cancer, brain cancer (glioma), uterine cancer, ovarian cancer, testicular cancer, leukemia, lymphoma, liver cancer, bladder cancer and kidney cancer. In another preferred embodiment, said disease or condition responsive to inhibition of MAGL is a neurodegenerative disease, neuroinflammation or amnesia, wherein said neurodegenerative disease is more preferably selected from the group consisting of: Alzheimer's disease, Parkinson's disease, multiple sclerosis, amyotrophic lateral sclerosis and traumatic brain injury (TBI). In another preferred embodiment, said disease or condition responsive to inhibition of MAGL is selected from the group consisting of: metabolic disease, diabetes, obesity and liver steatosis. In another preferred embodiment, said disease or condition responsive to inhibition of MAGL is selected from the group consisting of: anxiety, depression, stress, nausea, drug withdrawal and drug addiction, more preferably anxiety, depression or drug withdrawal, still more preferably drug withdrawal, wherein said drug withdrawal is selected from the group consisting of: nicotine withdrawal, cannabinoid withdrawal and opioid withdrawal. In another preferred embodiment, said disease or condition responsive to inhibition of MAGL is selected from the group consisting of: convulsions, seizures and epilepsy.

An especially preferred embodiment of the present invention relates to a compound of the formula (I), or a solvate or co-crystal thereof, for the treatment or prevention of a disease or condition selected from the group consisting of: pain, cancer, neurodegeneration, neuroinflammation, metabolic disease, obesity, diabetes, anxiety and depression, wherein:
- R is selected from the group consisting of: -CH₃, -CH₂-CH₂-CH=C(CH₃)₂, -CH₂-CH₂-CH=C(CH₃)-CH₂-CH₂-CH=C(CH₃)₂ and -CH₂-CH₂-CH=C(CH₃)-CH₂-CH₂-CH=C(CH₃)-CH₂-CH₂-CH=C(CH₃)₂;
- R₁ is selected from the group consisting of: -CH₃, -CH₂-CH₂-CH₃ and -CH₂-CH₂-CH₂-CH₂-CH₃;
- R₂ is selected from the group consisting of: H and CH₃;
- R₃ is selected from the group consisting of: H and CH₃; and
- R₄ is selected from the group consisting of: H, CH₃, and a benzyl moiety,
and wherein R₂ and R₃ are the same

The present invention also relates to a pharmaceutical or nutraceutical composition comprising a compound of the formula (I), or a solvate or co-crystal thereof, and at least one excipient. Said excipient is preferably selected from the group consisting of: binders, fillers, disintegrators, lubricants, coaters, sweeteners, flavourings, colourings, carriers, antioxidants, compactors and stabilisers. More preferably, two or more of said excipients are used in combination. One of skill in the art will recognize that particular excipients may have two or more functions in the oral dosage form.

The pharmaceutical or nutraceutical composition of the present invention can take the form of solutions, suspensions, emulsions, drops, tablets, pills, pellets, capsules, capsules containing liquids, powders, sustained-release formulations, suppositories, emulsions, aerosols, sprays, suspensions, or any other form suitable for administration. Thus, the composition described herein may be comprised in a capsule, tablet, pill, caplet, bottle, ampoule, sachet, syringe, cartridge, nebulizer or other container. In one embodiment, the pharmaceutical or nutraceutical composition is in the form of a capsule. In another embodiment, the pharmaceutical or nutraceutical composition is in the form of a tablet.

In the method of treatment or prevention of a disease or condition in a subject of the present invention, the administration of the compound of the formula (I), or a solvate or co-crystal thereof, or a pharmaceutical composition thereof, is preferably selected from the group consisting of: oral and parenteral administration. Preferably, said administration is selected from the group consisting of: oral, intradermal, intramuscular, intraperitoneal, intravenous, subcutaneous, intranasal, epidural, sublingual, intranasal, intracerebral, intrahepatic, intrapancreatic, intravesicular, intravaginal, transdermal, rectally, by inhalation, or topical administration. The mode of administration is left to the discretion of the practitioner and depends in part upon the site of the medical condition. Preferably, administration results in the release of said compound of the formula (I), or a solvate or co-crystal thereof, into the bloodstream or lymphatic system, or both.

Dosage forms for oral administration include pharmaceutically acceptable solutions, admixtures, suspensions, dispersions and emulsions. Dosage forms suitable for parenteral administration (e.g. intravenous, intramuscular, intrahepatic, intrapancreatic, intraperitoneal, subcutaneous and intra-articular injection and infusion) include solutions, suspensions, dispersions and emulsions.

The compound of the formula (I), or a solvate or co-crystal thereof, and a pharmaceutical or nutraceutical composition thereof can be administered by controlled-release or sustained-release means or by delivery devices. Such dosage forms can be useful for providing controlled-release or sustained-release using, for example, hydropropyl cellulose, hydropropylmethyl cellulose, polyvinylpyrrolidone, Eudragit, other polymer matrices, gels, permeable membranes, osmotic systems, multilayer coatings, microparticles, liposomes, microspheres, or a combination thereof to provide the desired release profile in varying proportions. The invention thus provides single unit dosage forms suitable for oral administration such as tablets, capsules, gelcaps and caplets that are adapted for controlled-release or sustained-release.

It will be appreciated that the dose of the compound of the formula (I), or a solvate or co-crystal thereof, and a pharmaceutical or nutraceutical composition thereof, to be administered may vary according to the particular compound, the particular dosage form, and the mode of administration. In addition, the dose will depend upon pharmacodynamic and pharmacokinetic factors that may modify the effect of the compound of the formula (I), or a solvate or co-crystal thereof, (e.g. body weight, gender, diet, time of administration, route of administration, rate of excretion, condition of the subject, drug combinations, genetic disposition and reaction sensitivities).

Individual doses of the compound of the formula (I), or a solvate or co-crystal thereof, can be administered in unit dosage forms containing, for example, from 0.01 mg to 5000 mg. Preferably, the compound of the formula (I), or a solvate or co-crystal thereof, is administered at an amount of from 0.1 mg to 1000 mg daily. More preferably, a suitable dosage of the compound of the formula (I), or a solvate or co-crystal thereof, is in a range of about 0.01 mg/kg to about 100 mg/kg of body weight of the subject, still more preferably in a range of about 0.05 mg/kg to about 10 mg/kg of body weight.

In accordance with certain embodiments of the invention, the compound of the formula (I), or a solvate or co-crystal thereof, may be administered once per day, more than once per day, every second day, every third day, once per week, once every two weeks, once every month, once every two months, once every three months, once every six months, or once per year.

### Examples

The following examples illustrate the invention and should not be considered as limiting, but rather representative of the invention.

### Example 1: Syntheses

The syntheses of the following compounds of the present invention is described in the present example:
2-methyl-2-(4-methylpent-3-enyl)-7-pentyl-5-chromenol [(A'), cannabichromene, CBC];
2,2-dimethyl-7-pentyl-5-chromenol [(B'), de(prenyl)-CBC];
2-methyl-2-(4-methylpent-3-enyl)-7-propyl-5-chromenol [(C'), cannabivarichromene, CBVC];
2,6-dimethyl-2-(4-methylpent-3-enyl)-7-pentyl-5-chromenol [(D'), 2-methylcannabichromene, 2-methyl CBC];
2-methyl-2-(4,8-dimethyl-nona-3,7-dienyl)-7-pentyl-5-chromenol [(E'), prenyl-CBC];
2-methyl-2-(4,8,12-trimethyl-trideca-3,7,11-trienyl)-7-pentyl-5-chromenol [(F'), bis(prenyl)-CBC];
2-methyl-2-(4-methylpent-3-enyl)-7-(1,1-dimethylheptyl)-5-chromenol [(G'), dimethylheptyl-CBC, DMH-CBC];
2-methyl-2-(4-methyl-penta-3-enyl)-7-pentyl-6-phenylmethyl-5-chromenol [(H'), 2-benzyl-CBC];
2-methyl-2-(4-methylpent-3-enyl)-7-pentyl-chromene-5,8-dione [(A), CBC-Quinol];
2,2-dimethyl-7-pentyl-chromene-5,8-dione [(B), de(prenyl)-CBC-Quinol];
2-methyl-2-(4-methylpent-3-enyl)-7-propyl-chromene-5,8-dione [(C), CBCV-Quinol];
2,6-dimethyl-2-(4-methylpent-3-enyl)-7-pentyl-chromene-5,8-dione [(D), 2-methyl-CBC-Quinol];
2-methyl-2-(4,8-dimethyl-nona-3,7-dienyl)-7-pentyl-chromene-5,8-dione [(E), prenyl-CBC-Quinol];
2-methyl-2-(4,8,12-trimethyl-trideca-3,7,11-trienyl)-7-pentyl-chromene-5,8-dione [(F), bis(prenyl)-CBC-Quinol];
2-methyl-2-(4-methylpent-3-enyl)-7-(1,1-dimethylheptyl)-chromene-5,8-dione [(G), DMH-CBC-Quinol];
2-methyl-2-(4-methyl-penta-3-enyl)-7-pentyl-6-phenylmethyl-chromene-5,8-dione [(H), 2-benzyl-CBC-Quinol];
2-methyl-2-(4-methylpent-3-enyl)-6-(4-morpholinylmethyl)-7-pentyl-5-chromenol (2-morpholinomethyl CBC); and
2-methyl-2-(4-methylpent-3-enyl)-6-[4-morpholinyl(phenyl)methyl]-7-pentyl-5-chromenol (2-morpholinobenzyl CBC).

### 1. Synthesis of chromenic phytocannabinoids by base-catalysed condensation.

### Synthesis of 2-methyl-2-(4-methylpent-3-enyl)-7-(1,1-dimethylheptyl)-5-chromenol (G')

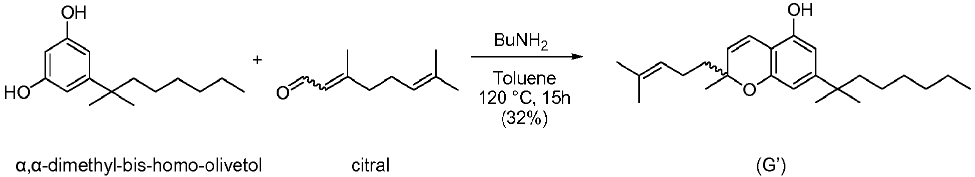

To a stirred solution of α,α-dimethyl-bis-homo-olivetol (TCI Chemical, 0.350 g, 1.48 mmol, 1.04 mol equivalents) in dry toluene was added BuNH₂ (141 µL, 1.42 mmol, 1 mol equivalent) and the mixture was heated for 15 minutes at 60 °C (oil bath temperature). Then, citral (Sigma Aldrich, 244 µL, 1.42 mmol, 1 mol equivalent) was added dropwise and the reaction was heated to 120 °C (oil bath temperature) for 15 hours. The resulting mixture was quenched with an aqueous solution of NaOH 1% and diluted with ethyl acetate. The organic phase was washed with brine, dried and evaporated. The crude product was purified by GCC (petroleum ether/CH₂Cl₂ in a 95:5 volume/volume ratio as eluant) to afford 2-methyl-2-(4-methylpent-3-enyl)-7-(1,1-dimethylheptyl)-5-chromenol [(G'), DMH-CBC, 7-depentyl-7-(1,1-dimethylheptyl) cannabichromene] (0.175 g, 32%) as a brown oil.

The same protocol was used for the synthesis of (A'), (B'), (C'), (E') and (F'), wherein in the synthesis of:
(A'), olivetol (Sigma Aldrich) replaces α,α-dimethyl-bis-homo-olivetol;
(B'), prenyl aldehyde (Sigma Aldrich) replaces citral and olivetol replaces α,α-dimethyl-bis-homo-olivetol;
(C'), divarin (Toronto Research Chemicals) replaces α,α-dimethyl-bis-homo-olivetol;
(E'), farnesal (Sigma Aldrich) replaces citral and olivetol replaces α,α-dimethyl-bis-homo-olivetol;
(F'), geranylgeranial (Yuhao Chemical) replaces citral and olivetol replaces α,α-dimethyl-bis-homo-olivetol; at the molar equivalents used in the aforementioned synthesis of (G').

### 2. Oxidation of chromenic phytocannabinoids.

### Oxidation of 2-methyl-2-(4-methylpent-3-enyl)-7-(1,1-dimethylheptyl)-5-chromenol (G')

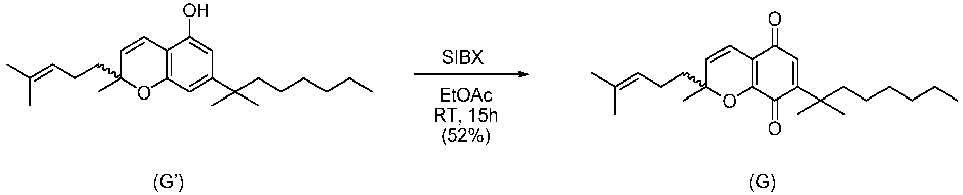

To a solution of 7-depentyl-7-(1,1-dimethylheptyl)cannabichromene (G') (0.067 g, 1.80 mmol, 1 mol equivalent) in ethyl acetate, SIBX was added (0.168 g, 0.60 mmol, 3.33 mol equivalent), and the mixture was stirred at between 20 and 25 °C for 15 hours, over which time the suspension slowly turned from pale brown to a strong purple colour. The reaction mixture was filtered and the solid was washed with ethyl acetate. The white solid filtrand was discarded and the purple filtrate was concentrated under reduced pressure. The crude product was purified by GCC (petroleum ether/CH₂Cl₂ in a 8:2 volume/volume ratio as eluant) to afford 2-methyl-2-(4-methylpent-3-enyl)-7-(1,1-dimethylheptyl)-chromene-5,8-dione [(G), DMH-CBC-Quinol, 7-depentyl-7-(1,1-dimethylheptyl) cannabichromene quinone] (0.040 g, 52%) as a red oil.

The same protocol was used for the synthesis of (A), (B), (C), (D), (E), (F) and (H), wherein in the synthesis of:
(A), cannabichromene (A') replaces 2-methyl-2-(4-methylpent-3-enyl)-7-(1,1-dimethylheptyl)-5-chromenol;
(B), 2,2-dimethyl-7-pentyl-5-chromenol (B') replaces 2-methyl-2-(4-methylpent-3-enyl)-7-(1,1-dimethylheptyl)-5-chromenol;
(C), cannabivarichromene (C') replaces 2-methyl-2-(4-methylpent-3-enyl)-7-(1,1-dimethylheptyl)-5-chromenol;
(D), 2-methylcannabichromene (D') replaces 2-methyl-2-(4-methylpent-3-enyl)-7-(1,1-dimethylheptyl)-5-chromenol;
(E), 2-methyl-2-(4,8-dimethyl-nona-3,7-dienyl)-7-pentyl-5-chromenol (E') replaces 2-methyl-2-(4-methylpent-3-enyl)-7-(1,1-dimethylheptyl)-5-chromenol;
(F), 2-methyl-2-(4,8,12-trimethyl-trideca-3,7,11-trienyl)-7-pentyl-5-chromenol (F') replaces 2-methyl-2-(4-methylpent-3-enyl)-7-(1,1-dimethylheptyl)-5-chromenol;
(H), 2-methyl-2-(4-methyl-penta-3-enyl)-7-pentyl-6-phenylmethyl-5-chromenol (H') replaces 2-methyl-2-(4-methylpent-3-enyl)-7-(1,1-dimethylheptyl)-5-chromenol;
at the molar equivalents used in the aforementioned synthesis of (G).

### 3. Mannich reaction.

### Preparation of 2-methyl-2-(4-methylpent-3-enyl)-6-(4-morpholinylmethyl)-7-pentyl-5-chromenol (2-morpholinomethyl CBC) from cannabichromene (A')

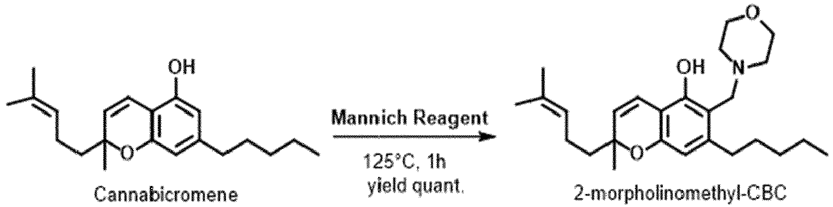

The Mannich reagent derived from morpholine (Sigma Aldrich) and paraformaldehyde (Sigma Aldrich, 50 mg; molecular weight ∼87.26 g/mol.; 0.573 mmol.; 1 equivalent) was added to CBC (200 mg; molecular weight 314.46 g/mol; 0.1636 mmol; 1 equivalent; silica gel TLC, *R*_{f}: 0.49 in petroleum ether/ethyl acetate in a 9:1 volume/volume ratio as eluant, UV 254 nm) and the resulting solution was stirred at room temperature (20 °C). The mixture was heated to 125 °C for 1 hour, while monitoring the course of reaction by TLC. The reaction mixture was worked up by dilution with brine and extraction of the aqueous phase with ethyl acetate. The organic phase was washed with brine until the washing solution became neutral, dried with Na₂SO₄, filtered and the solvent evaporated. The residue was purified by column chromatography (petroleum ether/ethyl acetate in a 9:1 volume/volume ratio as eluant and 10 mL of silica gel as stationary phase) to afford an amber oil (180 mg, quantitative yield) identified as 2-methyl-2-(4-methylpent-3-enyl)-6-(4-morpholinylmethyl)-7-pentyl-5-chromenol (2-morpholinomethyl CBC) (silica gel TLC, *R*_{f}: 0.42 in petroleum ether/ethyl acetate in a 9:1 volume/volume ratio as eluant, UV 254 nm; Dragendorff revelation).

The same protocol was used for the synthesis of 2-methyl-2-(4-methylpent-3-enyl)-6-[4-morpholinyl(phenyl)methyl]-7-pentyl-5-chromenol (2-morpholinobenzyl CBC), wherein benzaldehyde (Sigma Aldrich) replaces paraformaldehyde at the molar equivalent used in the aforementioned synthesis of 2-methyl-2-(4-methylpent-3-enyl)-6-(4-morpholinylmethyl)-7-pentyl-5-chromenol.

### 4. Synthesis of chromenic phytocannabinoids by reductive deamination

### Synthesis of 2,6-dimethyl-2-(4-methylpent-3-enyl)-7-pentyl-5-chromenol (D')

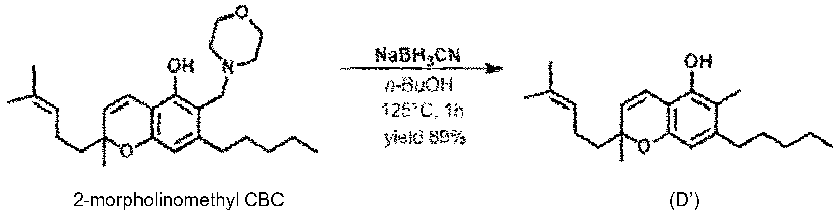

To a solution of 2-methyl-2-(4-methylpent-3-enyl)-6-(4-morpholinylmethyl)-7-pentyl-5-chromenol (2-morpholinomethyl CBC) (170 mg; molecular weight 413.59 g/mol.; 0.411 mmol.; 1 equivalent; silica gel TLC *R*_{f}: 0.42 in petroleum ether/ethyl acetate in a 9:1 volume/volume ratio as eluant; UV 254 nm) dissolved in *n*-butanol (4 mL), NaBH₃CN (Sigma Aldrich, 26 mg; molecular weight 62.84 g/mol; 0.411 mmol.; 1 equivalent) was added under continuous stirring at room temperature. The mixture was heated to 120 °C for 1 hour monitoring the course of reaction by TLC. The reaction was worked up by cooling at room temperature, dilution with brine and extraction with diethyl ether. The organic phase was washed three times with brine (3 x 20 mL), dried with Na₂SO₄, filtered and the solvent evaporated. The residue was purified by column chromatography (petroleum ether/ethyl acetate in a 9:1 volume/volume ratio as eluant and 10 mL of silica gel as stationary phase) to afford an amber oil (120 mg, yield 89%) identified as 2,6-dimethyl-2-(4-methylpent-3-enyl)-7-pentyl-5-chromenol [(D'), 2-methyl CBC, 2-methylcannabichromene] (silica gel TLC *R*_{f}: 0.54 in petroleum ether/ethyl acetate in a 9:1 volume/volume ratio as eluant; UV 254 nm).

The same protocol was used for the synthesis of 2-methyl-2-(4-methylpent-3-enyl)-7-pentyl-6-phenylmethyl-5-chromenol (H'), wherein 2-methyl-2-(4-methylpent-3-enyl)-6-[4-morpholinyl(phenyl)methyl]-7-pentyl-5-chromenol (2-morpholinobenzyl CBC) replaces 2-methyl-2-(4-methylpent-3-enyl)-6-(4-morpholinylmethyl)-7-pentyl-5-chromenol (2-morpholinomethyl CBC).

### Example 2: In vitro inhibition of mono-acyl glycerol lipase

The ability of the compounds of the present invention to inhibit mono-acyl glycerol lipase (MAGL) was estimated using their IC₅₀ value. MALG inhibition was measured using the Monoacylglycerol Lipase Inhibitor Screening Assay Kit (Cayman Chemical, USA). Briefly, in 96-well plate, samples and human recombinant enzyme were incubated for five minutes at room temperature (20 °C). After that, an ethanolic solution of 4-nitrophenylacetate (236 µM), as colorimetric substrate, was added to the wells and plates were incubated for 30 minutes at room temperature. Finally, absorbance was measured at 412 nm. JZL195 {(4-nitrophenyl)-4-[(3-phenoxyphenyl)methyl]piperazine-1-carboxylate, 5 µM} was used as a positive control. Background obtained was subtracted in each experimental value and the percentage of inhibition was established with the formula (Absorbance 100% Activity - Absorbance of the sample)/Absorbance 100% Activity. The IC₅₀ values for each of compounds (A), (B) and (C) against MAGL are shown in Table 1.

**Table 1: MAGL inhibition (IC₅₀) for compounds of the invention**

| **Compound** | | **IC₅₀ against MAGL (µM)** |
|---|---|---|
| **Reference** | **Structure** | |
| (A) | 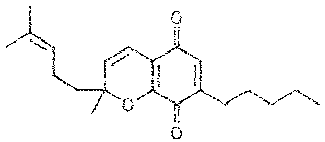 | 4.44 |
| (B) | 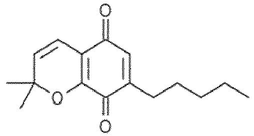 | 4.50 |
| (C) | 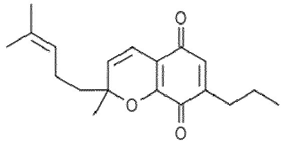 | 6.64 |

Thus, the compounds of the present invention effectively inhibit mono-acyl glycerol lipase (MAGL).

## Claims

1. A compound of the formula (I) or a solvate or co-crystal thereof 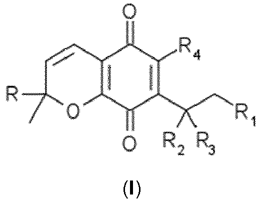 wherein:
- R is selected from the group consisting of: a C₁-C₆ alkyl, a C₂-C₈ alkenyl moiety, a C₄-C₁₂ dienyl or a C₆-C₁₈ trienyl moiety;
- R₁ is a C₁-C₆ alkyl moiety;
- R₂ is selected from the group consisting of: H and a C₁-C₆ alkyl moiety;
- R₃ is selected from the group consisting of: H and a C₁-C₆ alkyl moiety;
- R₄ is selected from the group consisting of: H, a C₁-C₆ alkyl moiety and a benzyl moiety.

2. The compound according to claim 1, wherein
- R is selected from the group consisting of: a C₁-C₆ alkyl, a C₄-C₈ alkenyl moiety, a C₈-C₁₂ dienyl or a C₁₄-C₁₈ trienyl moiety;
- R₁ is a C₁-C₅ alkyl moiety;
- R₂ is selected from the group consisting of: H and a C₁-C₆ alkyl moiety;
- R₃ is selected from the group consisting of: H and a C₁-C₆ alkyl moiety; and
- R₄ is selected from the group consisting of: H, a C₁-C₆ alkyl moiety and a benzyl moiety,
and wherein R₂ and R₃ are the same.

3. The compound according to any of claims 1 or 2, wherein
- R is selected from the group consisting of: -CH₃, -CH₂-CH₂-CH=C(CH₃)₂, -CH₂-CH₂-CH=C(CH₃)-CH₂-CH₂-CH=C(CH₃)₂ and -CH₂-CH₂-CH=C(CH₃)-CH₂-CH₂-CH=C(CH₃)-CH₂-CH₂-CH=C(CH₃)₂;
- R₁ is selected from the group consisting of: -CH₃ and -CH₂-CH₂-CH₃.
- R₂ is selected from the group consisting of: H and CH₃;
- R₃ is selected from the group consisting of: H and CH₃; and
- R₄ is selected from the group consisting of: H, CH₃ and a benzyl moiety,
and wherein R₂ and R₃ are the same.

4. The compound according to any of claims 1 to 3, wherein said compound of formula (I) is selected from the group consisting of: compounds (A), (B), (C), (D), (E), (F), (G) and (H):

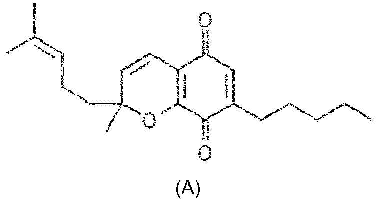

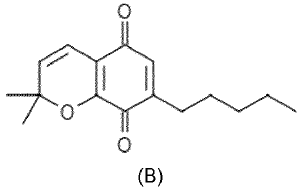

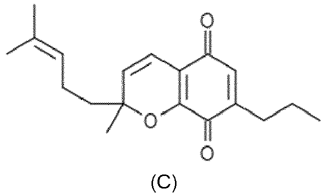

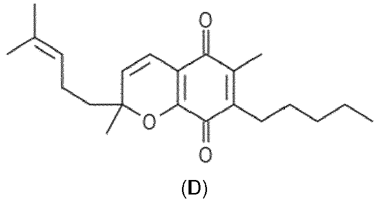

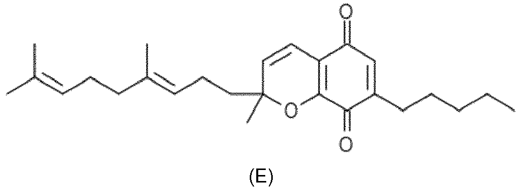

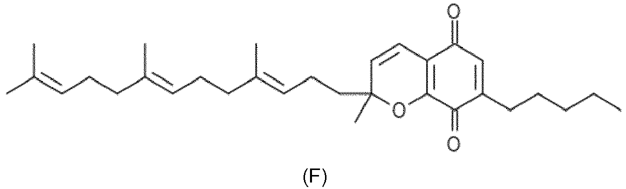

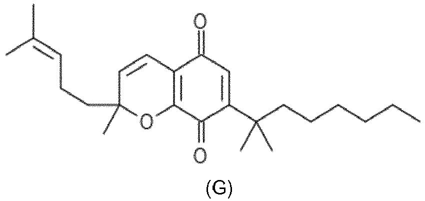

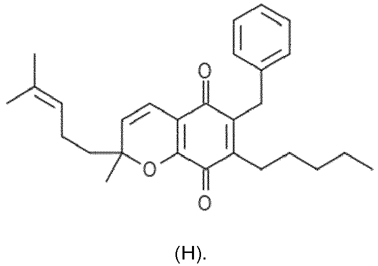

5. The compound according to any of claims 1 to 4, wherein said compound of formula (I) is selected from the group consisting of: compounds (A), (B) and (C):

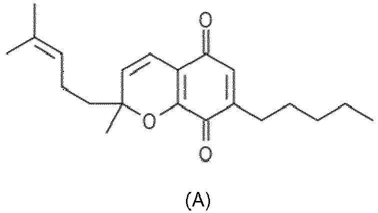

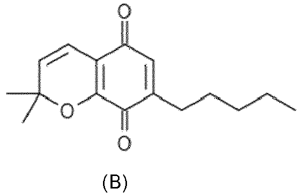

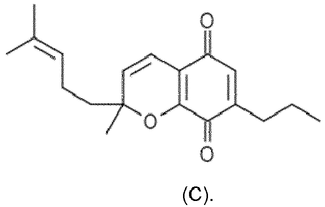

6. A process for producing a compound of the formula (I), or a solvate or co-crystal thereof 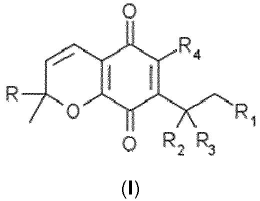 which comprises reacting a compound of formula (II), or a salt, solvate or co-crystal thereof 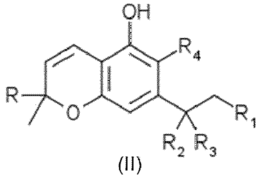 with a hypervalent iodine compound,
wherein:
- R is selected from the group consisting of: a C₁-C₆ alkyl, a C₂-C₈ alkenyl moiety, a C₄-C₁₂ dienyl or a C₆-C₁₈ trienyl moiety;
- R₁ is a C₁-C₆ alkyl moiety;
- R₂ is selected from the group consisting of: H and a C₁-C₆ alkyl moiety;
- R₃ is selected from the group consisting of: H and a C₁-C₆ alkyl moiety;
- R₄ is selected from the group consisting of: H, a C₁-C₆ alkyl moiety and a benzyl moiety.

7. The process according to claim 6, wherein
- R is selected from the group consisting of: a C₁-C₆ alkyl, a C₄-C₈ alkenyl moiety, a C₈-C₁₂ dienyl or a C₁₄-C₁₈ trienyl moiety;
- R₁ is a C₁-C₅ alkyl moiety;
- R₂ is selected from the group consisting of: H and a C₁-C₆ alkyl moiety;
- R₃ is selected from the group consisting of: H and a C₁-C₆ alkyl moiety; and
- R₄ is selected from the group consisting of: H, a C₁-C₆ alkyl moiety and a benzyl moiety,
and wherein R₂ and R₃ are the same.

8. The process according to any of claims 6 or 7, wherein
- R is selected from the group consisting of: -CH₃, -CH₂-CH₂-CH=C(CH₃)₂, -CH₂-CH₂-CH=C(CH₃)-CH₂-CH₂-CH=C(CH₃)₂ and -CH₂-CH₂-CH=C(CH₃)-CH₂-CH₂-CH=C(CH₃)-CH₂-CH₂-CH=C(CH₃)₂;
- R₁ is selected from the group consisting of: -CH₃ and -CH₂-CH₂-CH₃.
- R₂ is selected from the group consisting of: H and CH₃;
- R₃ is selected from the group consisting of: H and CH₃; and
- R₄ is selected from the group consisting of: H, CH₃ and a benzyl moiety,
and wherein R₂ and R₃ are the same.

9. The process according to any of claims 6 to 8, wherein said compound of formula (I) is selected from the group consisting of: compounds (A), (B), (C), (D), (E), (F), (G) and (H): 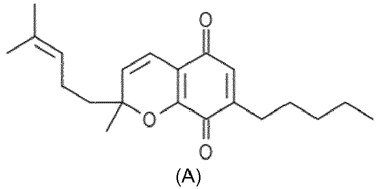 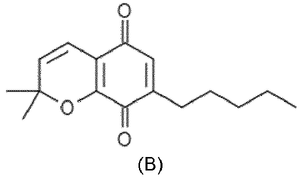 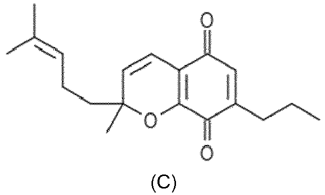 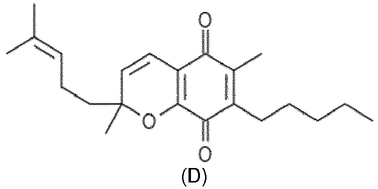 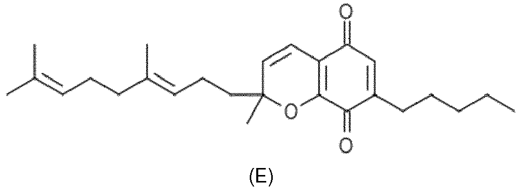 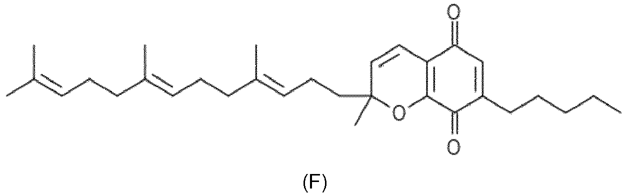 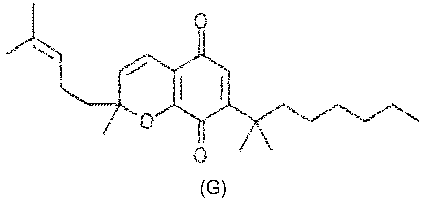 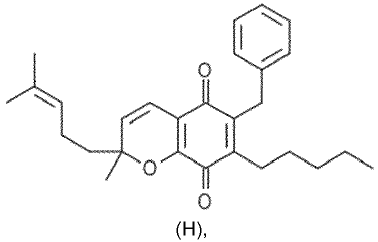 wherein:
- when said compound of formula (I) is compound (A), the compound of formula (II) is compound (A') 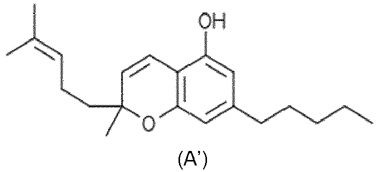
- when said compound of formula (I) is compound (B), the compound of formula (II) is compound (B') 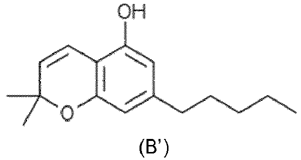
- when said compound of formula (I) is compound (C), the compound of formula (II) is compound (C') 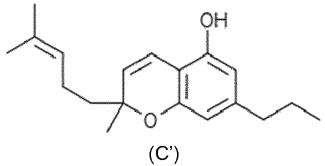
- when said compound of formula (I) is compound (D), the compound of formula (II) is compound (D') 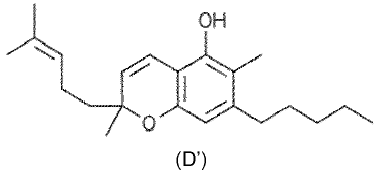
- when said compound of formula (I) is compound (E), the compound of formula (II) is compound (E') 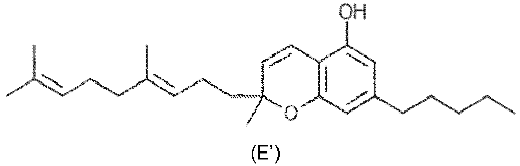
- when said compound of formula (I) is compound (F), the compound of formula (II) is compound (F') 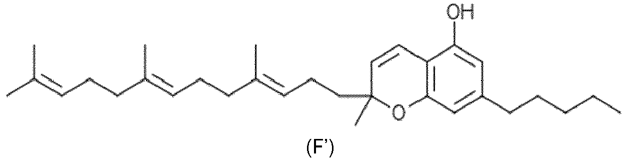
- when said compound of formula (I) is compound (G), the compound of formula (II) is compound (G') 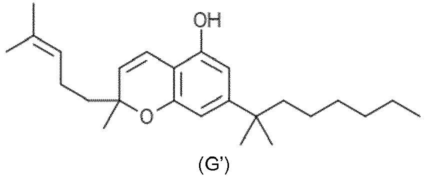
- when said compound of formula (I) is compound (H), the compound of formula (II) is compound (H')

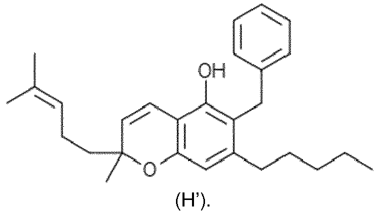

10. The process according to any of claims 6 to 9, wherein said compound of formula (I) is selected from the group consisting of: compounds (A), (B) and (C): 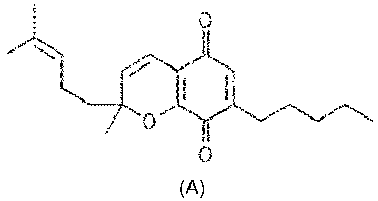 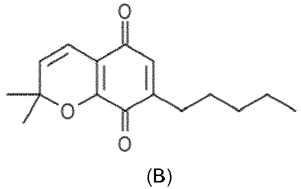 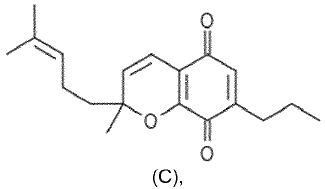 wherein:
- when said compound of formula (I) is compound (A), the compound of formula (II) is compound (A') 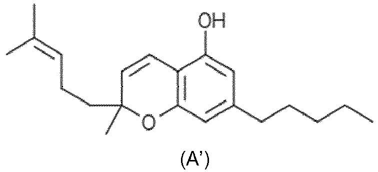
- when said compound of formula (I) is compound (B), the compound of formula (II) is compound (B') 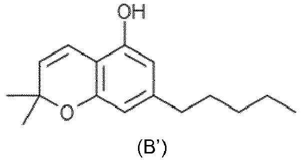
- when said compound of formula (I) is compound (C), the compound of formula (II) is compound (C')

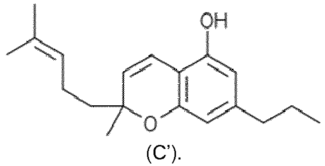

11. The process according to any of claims 6 to 10, wherein when R₄ is H, the compound of formula (II) is formed by reacting a compound of formula (III) 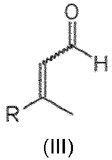 with a compound of formula (IV) 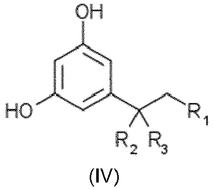 in the presence of a primary amine.

12. The process according to any of claims 6 to 10, wherein when R₄ is a C₁-C₆ alkyl moiety of the formula-CH₂-R₅ or a benzyl moiety of the formula -CH₂-R₅, the compound of formula (II) is formed by reacting a compound of formula (V) 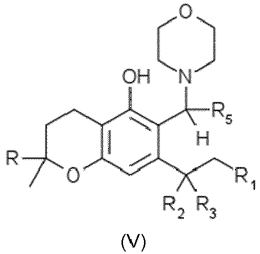 with a borohydride salt
wherein R₅ is selected from the group consisting of: H, a C₁-C₆ alkyl moiety and a phenyl moiety, and
- when R₅ is H, R₄ is a C₁ alkyl moiety;
- when R₅ is -CH₃, R₄ is a C₂ alkyl moiety;
- when R₅ is a C₂ alkyl moiety, R₄ is a C₃ alkyl moiety;
- when R₅ is a C₃ alkyl moiety, R₄ is a C₄ alkyl moiety;
- when R₅ is a C₄ alkyl moiety, R₄ is a C₅ alkyl moiety;
- when R₅ is a C₅ alkyl moiety, R₄ is a C₆ alkyl moiety; and
- when R₅ is a phenyl moiety, R₄ is a benzyl moiety.

13. The process according to claim 12, wherein said compound of formula (V) is formed by reacting a compound of formula (II) in which R₄ is H with a compound formed by reaction of morpholine with a compound of formula (VI) 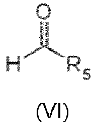 wherein R₅ is the same in the compound of formula (V) and in the compound of formula (VI).

14. Compound according to any of claims 1 to 5 for use in the treatment or prevention of a disease or condition.

15. Pharmaceutical composition comprising a compound according to any of claims 1 to 5 and at least one excipient.
